# EUROPEAN PATENT APPLICATION

(11) **EP 0 799 607 A2**
(43) Date of publication of application: **08.10.1997**
(21) Application number: 97301885.6
(22) Date of filing: 20.03.1997
(51) Int. Cl.: A61F 2/06

(54) **Intravascular stent having flattened profile**

(30) Priority: 01.04.1996 US 626705
(71) Applicant: MEDTRONIC, INC., Minneapolis, Minnesota 55432 (US)
(72) Inventor: Shearon, Lawrence W., Minnesota 55113 (US)
(74) Representative: Hughes, Andrea Michelle

(57) **Abstract**

A medical device and method of making which is used in supporting a luminal surface of a human or an animal body. The device comprises a stent mounted on the distal end of a catheter with the stent comprising a hollow cylindrical wire segment winding. The wire segment winding forms a plurality of spaced-apart elements each extending 360 degrees around the hollow cylinder. Each of the wire elements has a flattened cross-section which lies on the flat against the catheter throughout its length in an unexpanded diameter. Each of the elements has a plurality of extendible portions lying flat with respect to the cylinder which permit the wire elements to be expanded from the unexpanded diameter to a second, expanded diameter with the flattened cross-section of each of the wire elements lying flat against the luminal surface throughout its length. The medical device includes a means on the catheter for releasing the stent from the catheter in the expanded diameter.

## Description

The present invention relates to intravascular stent implants for maintaining vascular patency in humans and animals.

Percutaneous transluminal coronary angioplasty (PTCA) is used to reduce arterial build-up of cholesterol fats or atherosclerotic plaque. Typically a large guidewire of about 0.95mm (.038 inch) diameter is steered through the vascular system to the site of therapy. A guiding catheter, for example, can then be advanced over the guidewire to a point just proximal of the stenosis. The large guidewire is then removed. A balloon catheter on a smaller 0.35mm (.014 inch) guidewire is advanced within the guiding catheter to a point just proximal of the stenosis. The guidewire is advanced into the stenosis, followed by the balloon. The balloon at the distal end of the catheter is inflated causing the site of the stenosis to widen. The dilatation of the occlusion, however, can form flaps, fissures and dissections which threaten reclosure of the dilated vessel or even perforations in the vessel wall. Implantation of a metal stent can provide support for such flaps and dissections and thereby prevent reclosure of the vessel or provide a patch repair for a perforated vessel wall until corrective surgery can be performed. Reducing the possibility of restenosis after angioplasty reduces the likelihood that a secondary angioplasty procedure or a surgical bypass operation will be necessary.

An implanted prosthesis such as a stent can preclude additional procedures and maintain vascular patency by mechanically supporting dilated vessels to prevent vessel collapse. Stents can also be used to repair aneurysms, to support artificial vessels as liners of vessels or to repair dissections. Stents are suited to the treatment of any body lumen, including the vas deferens, ducts of the gallbladder, prostate gland, trachea, bronchus and liver.
The body lumens range in size from small coronary vessels to the 28 mm aortic vessel. The invention applies to acute and chronic closure or reclosure of body lumens.

A typical stent is a cylindrically shaped wire formed device intended to act as a permanent prosthesis. A typical stent 10 ranges from 5 mm to 50 mm in length. A stent is deployed in a body lumen from a radially compressed configuration into a radially expanded configuration which allows it to contact and support a body lumen. The stent can be made to be radially self-expanding or expandable by the use of an expansion device. The self expanding stent is made from a resilient elastic material while the device expandable stent is made from a material which is plastically deformable. A plastically deformable stent can be implanted during a single angioplasty procedure by using a balloon catheter bearing a stent which has been crimped onto the balloon.
Deployment is effected after the stent has been introduced percutaneously, transported transluminally and positioned at a desired location by means of the balloon catheter. Stents radially expand as the balloon is inflated, forcing the stent into contact with the body lumen thereby forming a supporting structure for the vessel walls.

The 1.25cm (half-inch) biocompatible metal stent props open blocked coronary arteries, keeping them from contracting after balloon angioplasty. A balloon of appropriate size and pressure is first used to open the lesion. An atherectomy device could alternatively be used to open the lesion. The process is repeated with a stent crimped on the balloon. The stent is deployed when the balloon is inflated. The stent remains as a permanent scaffold after the balloon is withdrawn.

U.S. Patent No. 4,739,762 to **Palmaz** employs flattened elements made from a tube. The elements lie flat with respect to the delivery catheter. During expansion of the stent, however, some of the flattened elements can twist away from their initial tubular profile such that when the stent is fully expanded, the flattened portion of the elements face the luminal flow. This is undesirable where blood may be flowing since the disruption of blood flow can lead to clot formation and closure of luminal flow.

Various shapes of stents are known in the art. U.S. Patent No. 4,886,062 to **Wiktor** for "Intravascular Radially Expandable Stent and Method of Implant" discloses a two-dimensional zig-zag form, typically a sinusoidal form. A typical Wiktor coronary stent is formed with a wire segment which is formed of a round wire into a sinusoidal wave form helix pattern the length of the stent by a means such as passing the wire through gears such as disclosed in U.S. Patent 2,153,936 issued to **Owens** et al. The wire segment is wound around a forming mandrel then manually reduced in diameter by being pressed around successively smaller mandrels.

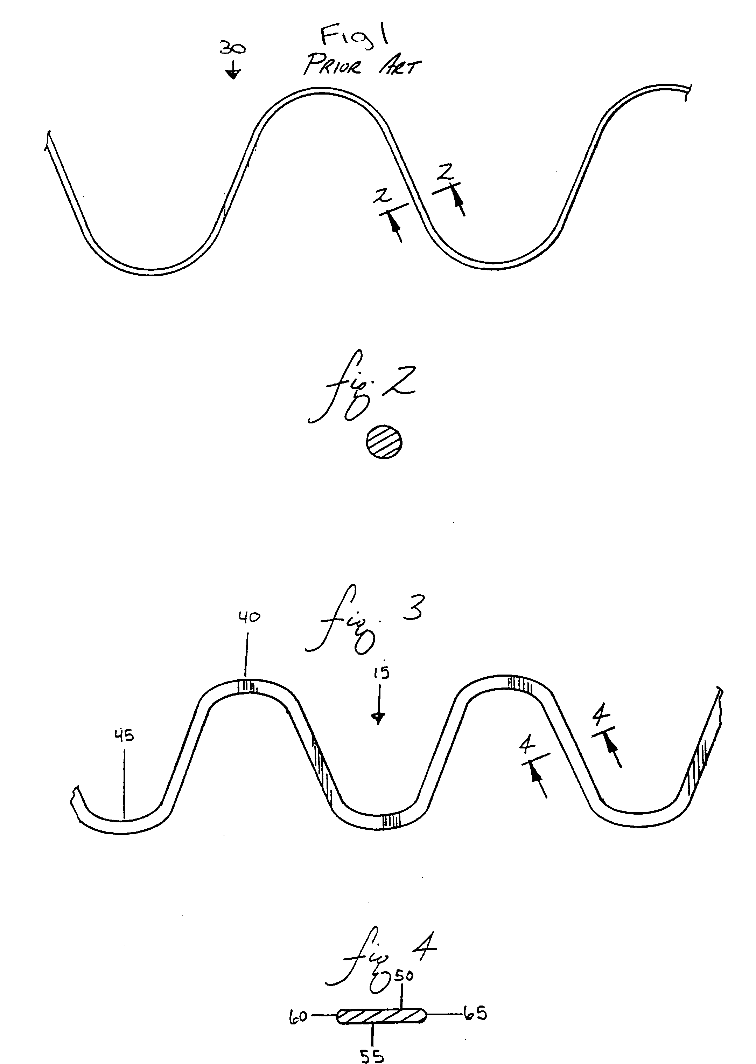

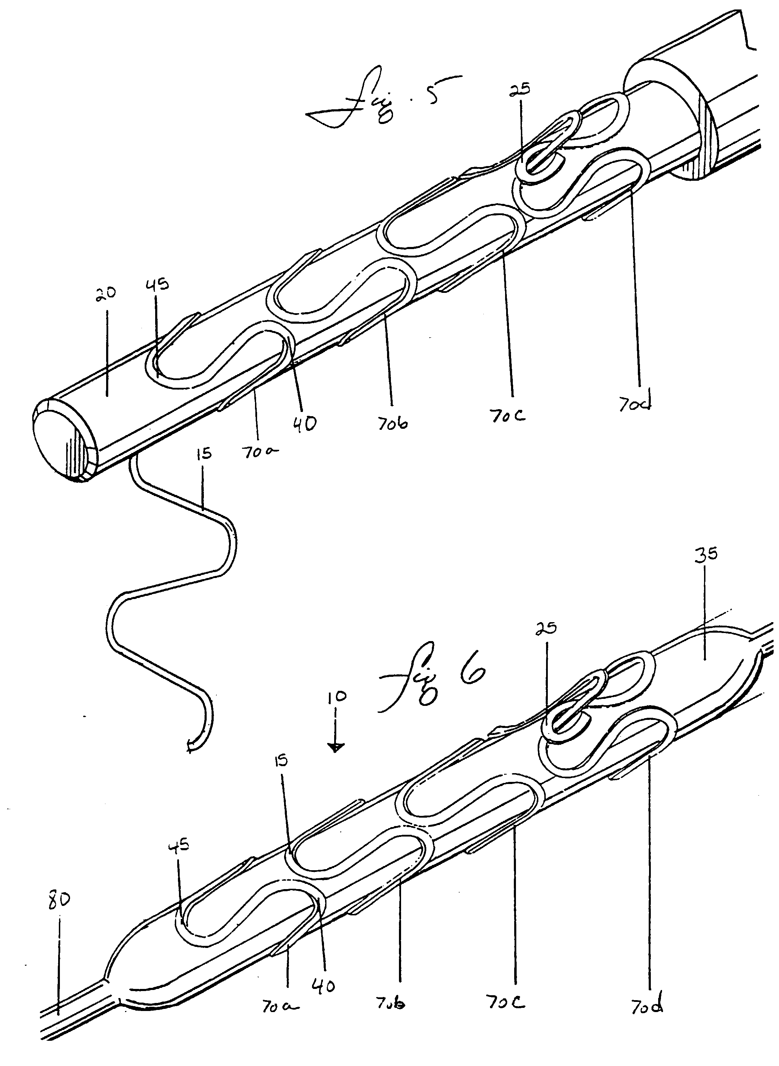

U.S. Patent Nos. 5,019,090 and 5,092,877 to **Pinchuk** for "Radially Expandable Endoprosthesis and the Like" disclose a plurality of adjacent generally circumferential sections that are substantially axially positioned with respect to each other. At least one of the generally circumferential sections has a generally circumferentially disposed expandable segment that imparts circumferential and radial expandability to the stent. In Fig. 2 the wound strand is subjected to flattening forces so that the three-dimensional wound strand is transformed into a generally planar shape.

U.S. Patent No. 5,222,971 to **Willard** et al. for "Temporary Stent and Method for use and Manufacture" discloses a flat wire of rectangular cross section. The edges formed on the flat wire are rounded off. The preferred method used to radius the corners of the flat wire is electropolishing which removes edges or protrusions of the material and passivates the metal without altering the bulk properties of the metal.

A text on *Coronary Stents* edited by U. **Sigwart** and G.I **Frank** on page 174 discloses a stent with a trapezoidal shaped wire. The long side faces the vessel and the short side faces the balloon. Such a stent wire having corners less than 90 degrees would tend to catch or cause perforations during deployment.

It is an object of the invention to provide a wire wound stent having good surface coverage as well as to provide longitudinal support during expansion, reduce stent profile and reduce the likelihood of thrombus formation. Another object of the invention is to provide flattened elements which do not twist away from their initial tubular profile such that when the stent is fully expanded, the flattened portion of the elements do not face the luminal flow.

According to a first aspect, the present invention provides a medical device for use in supporting a luminal surface of a human or animal body comprising:
a catheter having a proximal end and a distal end;
a stent mounted on the distal end of the catheter, the stent comprising a hollow cylindrical wire segment winding, the wire segment winding forming a plurality of spaced-apart elements each extending 360 degrees around the hollow cylinder, each of the wire elements having a flattened cross-section which lies flat against the catheter throughout its length in an unexpanded diameter, and each of the elements having a plurality of extendible portions lying flat with respect to the cylinder which permit the wire elements to be expanded from the unexpanded diameter to a second, expanded diameter with the flattened cross-section of each of the wire elements lying flat against the luminal surface throughout its length, wherein the height of the wire segment is uniform throught the stent; and
a means on the catheter for releasing the stent from the catheter in the expanded diameter.

According to a second aspect, there is provided a method for making a medical device for providing support for a luminal surface of a human or animal body comprising:
providing a flattened wire segment such that the wire segment has a width and a height, the width has an upper width and an opposing lower width, the upper width being of equal length to the lower width, the width being greater than the height, the upper width and the lower width being substantially flat, the height having a first side and an opposing second side, the first and second height sides being of equal length and sufficiently radiused such that the luminal surface does not become perforated;
preforming the flattened wire segment into a hollow cylindrical wire segment winding, the winding forming a plurality of spaced-apart elements,
providing a catheter;
extending the catheter longitudinally through the hollow cylindrical wire segment winding, each of the elements having a flattened cross-section which lies on the flat against the catheter throughout its length, the wire having a proximal end and a distal end; and
providing a means for releasing the winding in expanded form such that, the lower width side continues to face radially inward away from the luminal surface.

Preferred embodiments of the invention will now be described, by way of example only, with reference to the accompanying drawings.
FIG. 1 is a top plan view of a prior art round wire;
FIG. 2 is a cross-sectional view thereof taken along line 2 - 2 in Fig. 1;
FIG. 3 is a top plan view showing a flat wire;
FIG. 4 is a cross-sectional view thereof taken along the line 4 - 4 in Fig. 3;
FIG. 5 is a perspective view of the wire according to FIG. 3 being wrapped with respect to the forming mandrel;
FIG. 6 is a perspective view showing an overall view of a stent fitted on a balloon catheter and using the wire of FIG. 3; and
FIG. 7 is a perspective view of the stent of FIG. 6 on a balloon in expanded form.

A typical prior art round wire coronary stent may be formed with a round wire segment 30 as in Figs. 1 and 2 and formed into a sinusoidal waveform helix pattern the length of the stent by a means such as passing the wire through gears such as disclosed in U.S. Patent 2,153,936 issued to Owens et al.

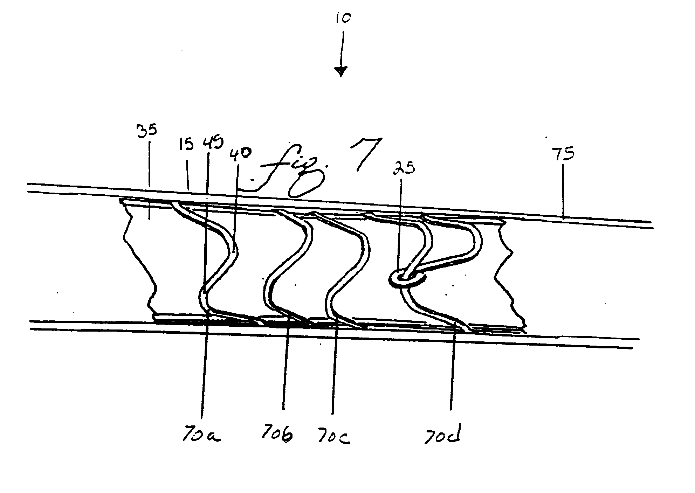

The present invention shown in Figs. 5 - 7, depicts a radially expandable stent 10 in the form of a hollow cylinder defined by a sequence of spaced apart wire elements 70a-d with each of the wire elements 70a-d extending 360 degrees around the cylinder. Each of the wire elements 70 has a flattened cross-section as seen in Fig. 4 which lies on the flat against the catheter 80 throughout its length in an expanded diameter. The wire elements 70a-d having extendible, sinusoidal zig-zags formed by smooth bends such as alternating peaks 40 and valleys 45 lying flat with respect to the cylinder and also with respect to the luminal surface 75 throughout its length. As shown, the peaks 40 and valleys 45 are shaped in a generally longitudinal direction along the cylinder at one point and then reverse their direction so that the peaks 40 and valleys 45 may open as the wire element 70a is expanded. Also as shown, the wire elements 70a-d are uniformly spaced along the cylinder and the peaks 40 and valleys 45 are uniformly spaced around the cylinder. The adjacent wire elements 70a-d are flexibly connected together in an end-to-end fashion by means of the helical winding of the flat wire segment 15. As shown in Figs. 5 -7, the wire elements 70a-d have a plurality of extendible portions, such as peaks 40 and valleys 45, lying flat with respect to the cylinder which permit the wire elements to be expanded from a first diameter as seen in Fig. 6 covering 360 degrees of the cylinder to a second, expanded diameter as seen in Fig. 7 covering 360 degrees of-the expanded cylinder. The flattened cross section of each of the wire elements 70a-d lay flat against the luminal surface 75 throughout its length.

The preferred form of the sinusoidal wave of either the round wire segment 30 or the flat wire segment 15 of this invention is that of a length of 12.5mm to 2.25mm (0.150 inches to 0.090 inches) and a wave amplitude of between 1.25mm to 2mm (0.050 inches and 0.080 inches). Any wave length and amplitude combination that would provide adequate vessel hoop strength and vessel coverage is appropriate, however. The stent 10 must expand evenly and permit the balloon 35 to expand evenly. The stent 10 and balloon 35 can be transported via a standard #8 French guiding catheter. Those skilled in the art would recognize that guiding catheters of other sizes can be used. Once on location, the stent 10 can be expanded radially by the expansion of the balloon 35; a ratio of 2.75:1 can be achieved with a round wire diameter of approximately 0.125mm (.005 inches) and an initial stent diameter of approximately 1.5mm (0.060 inches).

Flattening a round wire segment 30 yields a flat wire segment 15 with a lower profile with more radial and longitudinal strength as well as providing more surface area coverage with the vessel. The lower profile will also reduce blood turbulence thereby reducing the possibility of life-threatening thrombosis. Given the greater surface area contact of a flat wire stent, a higher pressure balloon may be beneficial to deploy such stents. A flattened round wire also yields radiused edges which reduces the likelihood of perforations.

The round wire segment 30 is first run through gears to form the sinusoidal shape, then the round wire segment 30 is flattened. A typical fixture may consist of two spur gears. The round wire segment 30 is formed into the sinusoidal shape by passing between the meshing gears as they are turned. With this type of fixture and a round wire there is no need to control the plane of the wire as it passes through the gears. Such a manufacturing process is relatively simple as compared to creating shapes, as for example, a trapezoidal shaped wire.

If the round wire segment 30 is first flattened then run through gears, the flat wire segment 15 would become twisted in the gears. Using a fixture with two spur gears on a flat wire 15 would flip the wire 90 degrees to the flat side as it went through the fixture.

The round wire segment 30 can be flattened by any conventional means, as for example, a hydraulic press. After flattening, the flat wire segment 15 has a width which is greater than the height. The width has an upper width side 50 and an opposing lower width side 55, the upper width side 50 faces radially outward toward the vessel wall. The lower width side 55 faces radially inward away from the vessel wall. The upper width side 50 and the lower width side 55 are of equal length and substantially flat. The height has a first side 60 and an opposing second side 65. The first and second height sides 60, 65 being of equal length and sufficiently radiused to prevent perforation of the vessel wall.

The standard 0.125mm (.005 inch) round wire segment 30 when flattened yields a flat wire segment 15 with a preferred width of approximately 0.175mm (.007 inches) and a preferred height of approximately 0.075mm (.003 inches) The preferred range for the width is between approximately 0.15mm (.006 inches) and approximately 0.25mm (.010 inches). Round wires of an approximately 0.125mm (.005 inch) diameter which are flattened to much wider than 0.25 to 0.3mm (.010 to .012 inches) could become razor thin and risk perforating the vessel wall. Wires with a width ranging from approximately 0.025mm (.001 inches) to approximately 0.125mm (.005 inches) are too thin and do not wrap properly around the forming mandrel; the wire must be cold worked (stressed) too much and will not form properly. The preferred range for the height is from approximately 0.0375mm (.0015 inches) to approximately 0.1mm (.004 inches) from a 0.125mm (.005 inch) diameter round wire.

As with a round wire 30, the flat wire segment 15 retains the peaks 40 and valleys 45 of the sinusoidal wave forms as it is wound around a forming mandrel 20 as in Fig. 5 to form a cylindrical shape. A forming mandrel sequence can provide a gradual reduction in the stent 10 outer diameter by applied finger pressure under microscopic observation. Although it is possible to go directly from a 3.75mm (0.150 inch) stent outer diameter to a 1.625mm (0.065 inch) stent outer diameter by placing stent 10 directly onto the balloon 35 from the forming mandrel and make an acceptable stent, it is more difficult to maintain proper alignment of the stent wires by doing so. It is preferred that the stent 10 is further processed from a 3.75mm (0.150 inch) diameter forming mandrel by pressing it onto a 2.5mm (0.100 inch) diameter forming mandrel, thereafter pressing it onto a 2mm (0.080 inch) diameter forming mandrel and finally pressing it onto a 1.625mm (0.065 inch) diameter forming mandrel before being applied to the balloon 35 as seen in FIG. 6. Those skilled in the art would recognize that a variety of acceptable mandrel sizes could be used in the forming sequence.

Given that the successive mandrel forming sequence requires repeated manual compression to successively and uniformly reduce the diameter of the stent, it was expected that a stent 10 made from a flat wire segment 15 may rotate and not lie flat in uniform contact with the vessel wall after balloon inflation. Somewhat unexpectedly, the flat surface of the stent remains in contact with the balloon upon balloon expansion as seen in FIG. 7, and therefore could be expected to remain in contact with the vessel wall.

A typical Wiktor stent terminates at the distal end and the proximal end with an end loop attachment 25 as seen in FIGS. 5 and 6. After the stent 10 has been reduced to the objective outer diameter, the proximal and distal ends of the wire segment 15 are manually looped around the nearest adjacent wave. When four, five or six waves per helical revolution are used, the proximal and distal ends of the wire segment 15 can be looped to the fourth, fifth or sixth respective wave back from either end of the wire segment 15. Somewhat unexpectedly, the end loop attachment 25 also stays flat against the balloon upon balloon expansion and therefore should stay flat against the vessel as well. Those skilled in the art will recognize other means of end attachments which may include twisting, biocompatible adhesive, brazing, crimping, welding or stamping.

The stent 10 is removed from the mandrel 20 and placed over a suitable expandable diameter device such as an inflatable balloon 35 which is typically used for angioplasty procedures. The stent 10 is centrally located and positioned with respect to the length of balloon 35. The stent 10 turns are evenly spaced so that when the stent 10 is expanded, the stent 10 will provide even support inside the vessel, and resist external loading. A stent can be implanted during a single angioplasty procedure by using a balloon catheter bearing a stent 10 which has been crimped with a suitable crimping tool (not shown) onto the balloon 35. Manually squeezing the stent 10 over the balloon 35 is also acceptable.

Once the balloon 35 is lodged in the stenosis, the balloon 35 can be inflated using standard angioplasty procedures and techniques. The stent 10 is thereby radially expanded as the balloon 35 is inflated, causing the stent 10 to contact the body lumen thereby forming a supporting relationship with the vessel wall luminal surface 75. As balloon 35 expands, so does stent 10. The expanding balloon 35 together with the stent 10 compresses the plaque in the stenosis and prevents possible reocclusion. When the angioplasty procedure is completed, balloon 35 is deflated and withdrawn leaving stent 10 firmly implanted within vessel. The previously occluded vessel is recannalized and patency is restored. Any protrusions are undesirable because they are conducive to turbulent blood flow and potential formation of thrombosis.

The balloon expandable stent 10 can be made of an inert, biocompatible material with high corrosion resistance that can be plastically deformed at low-moderate stress levels such as tantalum, the preferred embodiment. Other acceptable materials include nickel titanium, stainless steel, titanium ASTM F63-83 Grade 1, niobium or high carat gold K 19-22. A self-expanding device can be made by the use of superelastic NiTi such as nitinol manufactured by Raychem or Forukawa.

The preceding specific embodiments are illustrative of the practice of the invention. It is to be understood, however, that other expedients known to those skilled in the art or disclosed herein, may be employed without departing from the scope of the appended claims.

## Claims

1. A medical device for use in supporting a luminal surface of a human or animal body comprising:
a catheter (80) having a proximal end and a distal end;
a stent (10) mounted on the distal end of the catheter, the stent comprising a hollow cylindrical wire segment winding (15), the wire segment winding forming a plurality of spaced-apart elements (70a-d) each extending 360 degrees around the hollow cylinder, each of the wire elements having a flattened cross-section which lies flat against the catheter throughout its length in an unexpanded diameter, and each of the elements having a plurality of extendible portions (40,45) lying flat with respect to the cylinder which permit the wire elements to be expanded from the unexpanded diameter to a second, expanded diameter with the flattened cross-section of each of the wire elements lying flat against the luminal surface (75) throughout its length, wherein the height of the wire segment is uniform throught the stent; and
a means on the catheter for releasing the stent from the catheter in the expanded diameter.

2. The medical device according to claim 1 wherein the wire segment (15) has a height and a width, the width being greater than the height, the width having an upper width side and an opposing lower width side, the upper width side facing radially outward toward the luminal surface, the lower width side facing radially inward away from the luminal surface, the upper width side and the lower width side being substantially flat, the height having a first side and an opposing second side, the first and second height sides being pf equal length and sufficiently radiused such that the luminal surface does not become perforated.

3. The medical device according to claim 1 wherein the wire segment (15) has a height and a width, the width being greater than the height, the width having an upper width side and an opposing lower width side, the upper width side facing radially outward toward the luminal surface, the lower width side facing radially inward away from the luminal surface, the upper width side and the lower width side being of equal length and substantially flat, the height having a first side and an opposing second side, the first and second height sides being of equal length.

4. The medical device according to claim 1, 2 or 3 wherein the extendible portions include smooth bends.

5. The medical device according to claim 4 wherein the smooth bends are sinusoidal.

6. The medical device according to claim 1, wherein the wire segment is malleable, resilient and flat and wherein the elements are a preformed series of peaks (40) alternating with valleys (45) therein, the flat wire segment having a proximal end and a distal end;
the flat wire segment (15) being wound into a continuous helix having a hollow cylindrical shape;
the flat wire segment (15) having a height and a width, the width being greater than the height;
the width having an upper width side and an opposing lower width side, the upper width side facing radially outward toward the luminal surface, the lower width side facing radially inward away from the luminal surface, the upper width side and the lower width side being of equal length and substantially flat;
the height having a first side and an opposing second side, the first and second height sides being of equal length and sufficiently radiused such that the luminal surface does not become perforated; and
a means (35) for releasing the flat wire segment (15) in expanded form such that the lower width side continues to face radially inward away from the luminal surface.

7. The medical device according to claim 6 wherein the flat wire segment is formed of a biocompatible metal that can be plastically deformed at low to moderate stress levels.

8. The medical device according to claim 6 wherein the flat wire segment is formed of a super-elastic metallic material.

9. The medical device according to claim 6, 7 or 8 wherein the means for releasing the segment in expanded form comprises a balloon (35).

10. The medical device according to claim 6, 7, 8 or 9 wherein the wire segment has a width of approximately 0.175mm (.007 inches) and a height of approximately 0.075mm (.003 inches).

11. The medical device according to claim 6, 7, 8 or 9 wherein the wire has a width with a range of approximately 0.15mm to 0.25mm (.006 inches to .010 inches).

12. The medical device according to claim 6, 7, 8 or 9 wherein the wire has a height with a range of approximately 0.1mm to 0.375mm (.004 inches to .0015 inches).

13. A method for making a medical device for providing support for a luminal surface of a human or animal body comprising:
providing a flattened wire segment (15) such that the wire segment has a width and a height, the width has an upper width and an opposing lower width, the upper width being of equal length to the lower width, the width being greater than the height, the upper width and the lower width being substantially flat, the height having a first side and an opposing second side, the first and second height sides being of equal length and sufficiently radiused such that the luminal surface does not become perforated;
preforming the flattened wire segment into a hollow cylindrical wire segment winding, the winding forming a plurality of spaced-apart elements (70 a-d),
providing a catheter (80);
extending the catheter longitudinally through the hollow cylindrical wire segment winding, each of the elements having a flattened cross-section which lies on the flat against the catheter throughout its length, the wire having a proximal end and a distal end; and
providing a means (35) for releasing the winding in expanded form such that, the lower width side continues to face radially inward away from the luminal surface.

14. The method according to claim 13, wherein the step of providing said flattened wire segment comprises providing a round wire segment and flattening said round wire segment.

15. The method according to claim 13 or 14 including preforming the flattened wire segment into a sinusoidal wave pattern having a series of peaks (40) alternating with valleys (45), the wire having a proximal end- and a distal end.

16. The method according to claim 13, 14 or 15 wherein the means for releasing the winding in expanded form comprises a balloon (35).
